# EUROPEAN PATENT SPECIFICATION

(11) **EP 3 226 925 B1**
(45) Date of publication and mention of the grant of the patent: **23.06.2021**
(21) Application number: 15825788.1
(22) Date of filing: 02.12.2015
(51) Int. Cl.: A61L 29/08, A61L 29/14, A61L 29/16, A61L 31/10, A61L 31/14, A61L 31/16

(54) **DRUG ELUTING DEVICE**
ARZNEIMITTELELUTIONSVORRICHTUNG
DISPOSITIF D'ÉLUTION MÉDICAMENTEUSE

(30) Priority: 05.12.2014 GR 20140100628
(43) Date of publication of application: 11.10.2017
(73) Proprietor: Rontis Hellas S.A., 151 25 Marousi Athens (GR)
(72) Inventor: MOULAS, Anargyros, GR-412 22 Larisa (GR)
(86) International application number: PCT/EP2015/002427
(87) International publication number: WO 2016/091365

(56) References cited:
- EP-A1- 1 500 401
- WO-A2-2008/011093
- WO-A2-2010/003076
- None

## Description

### TECHNICAL FIELD OF THE INVENTION

The present invention relates to a drug-eluting device, and in particular to a drug eluting medical device, such as stent, balloon or graft, comprising an effective amount of retinoid receptor ligand for preventing or reducing restenosis and neointimal formation after vascular procedures, and a method for the preparation of said drug eluting device, and an improved method for the prevention of restenosis.

### BACKGROUND OF THE INVENTION

Percutaneous transluminal coronary angioplasty (PTCA) or coronary artery bypass graft (CABG) are the main options to treat blocked coronary arteries. PTCA is a procedure in which a small balloon catheter is passed down a blocked or narrowed coronary artery and then expanded to re-open the artery, thus eliminating the necessity of the more invasive surgical procedure of coronary artery bypass graft. A major complication with PTCA is the problem of post-angioplasty closure of the vessel, such as acute reocclusion or restenosis. To treat this condition, additional revascularization procedures are frequently required, thereby increasing trauma and risk to the patient.

Restenosis is a wound healing process that reduces the vessel lumen diameter by extracellular matrix deposition, neointimal hyperplasia, and vascular smooth muscle cell proliferation, and which may result in renarrowing or even reocclusion of the lumen.

Restenosis is commonly treated by a procedure known as stenting, wherein a medical device is surgically implanted in the affected artery to prevent it from occluding post procedure.

Coronary stents are typically used to reduce reocclusion of the artery. With the introduction of coronary stents, vascular dissections were stabilized and arterial recoil was eliminated, but neointimal accumulation remained problematic, resulting in the development of in-stent restenosis in 20% - 30% of cases.

A stent is typically cylindrical in shape and is usually made from a biocompatible metal, such as cobalt chromium or surgical steel. Most stents are collapsible and are delivered to the occluded artery via a translumenal catheter. The stent is affixed to the catheter and can be either self-expanding or expanded by inflation of a balloon inside the stent that is then removed with the catheter once the stent is in place.

Drug-eluting stents were developed to release antiproliferative agents at the site of arterial injury to attenuate neointimal formation. Drug-eluting stents typically comprise a metallic stent, a polymer-based drug delivery platform, and a pharmacologic agent such as an immunosuppressant and/or antiproliferative compound.

Various medical devices with certain agents are already known to reduce the complications that can arise from stent therapy including restenosis and thrombosis. However, the prior art has encountered substantial difficulties in the development of drug-eluting stents.

First-generation drug-eluting stents (DES), which impart the controlled release of sirolimus or paclitaxel from durable polymers to the vessel wall, have been consistently shown to reduce the risk of restenosis and target vessel revascularization compared with bare metal stents. These first-generation DES were associated with significant reductions in neointimal hyperplasia and in-stent restenosis however, the possibility of the drugs contributing to delayed arterial healing and late stent thrombosis could not be ignored. Stent thrombosis that emerged as a major safety concern with first-generation DES required prolonged dual antiplatelet therapy.

Second-generation DES using sirolimus analogues such as everolimus, zotarolimus, and biolimus have been developed to overcome these issues with improved stent designs and construction and the use of biocompatible and bioabsorbable polymers. However, the problem of thrombosis and even restenosis persists in a degree even with the latest generations of DESs. Although DESs have incrementally improved outcomes after percutaneous coronary intervention, delayed re-endothelialization and stent thrombosis remain important challenges.

WO 2010/003076 discloses a polymeric vascular stent, or a vascular graft, coated with a polymeric matrix comprising trans-retinoid acid. The polymeric matrix is a biodegradable polyester, an elastomer, or a hydrogel.

Moreover, all currently used drugs in DES include mainly cytostatic agents that do not occur naturally in the body, do not have a biological function under normal conditions and are in most cases cytotoxic, thus contributing to imperfect healing.

Although each of the above documents represents an attempt to overcome the complications associated with the use of implantable drug delivering medical devices, such as biocompatibility and drug release profile, there still exists a need for improving the efficacy and safety of drug eluting stents with the use of compounds and delivery methods that will reduce restenosis and thrombosis.

### SUMMARY OF THE INVENTION

It is, therefore, an object of the present invention to provide an improved drug eluting device for preventing and treating restenosis and neointimal formation after vascular procedures and/or implantation of implants, which overcomes the deficiencies of the prior art and avoids the cytotoxicity of previously used drugs thus leading to better healing and reduced adverse effects.

Moreover, it is another object of the present invention to provide a drug-eluting device, which comprises substances naturally occurring in the human body and their synthetic analogs in highly efficient doses of the active substance.

It is another object of the present invention to provide a drug-eluting device comprising an improved drug delivery system with the use of biodegradable polymers.

A further aspect of the present invention is to employ a suitable method for the preparation of a drug-eluting device for preventing and treating restenosis and neointimal formation after vascular procedures and/or implantation of implants, that is convenient, efficient, cost effective and by reducing the rate of occurrence and/or extent of restenosis or thrombosis.

Still another object of the present invention is to provide an improved method for reducing the rate of occurrence and/or extent of restenosis or thrombosis resulting from vascular procedures and/or implantation of implants in a subject, by using a drug eluting device coated with a polymeric release system.

In accordance with the above objects of the present invention, a drug-eluting device for preventing and treating restenosis and neointimal formation after vascular procedures and/or implantation of implants is provided as defined in claim 1.

According to another embodiment of the present invention, a process for the preparation of a drug eluting device for preventing and treating restenosis and neointimal formation after vascular procedures and/or implantation of implants comprising an implantable and non-implantable stent as defined in claim 3.

Moreover, according to another embodiment of the present disclosure, a method of preventing restenosis with a drug eluting device, is provided, comprising: providing a drug eluting stent for preventing and treating restenosis and neointimal formation after vascular procedures and/or implantation of implants comprising an implantable and non-implantable device, such as stent, balloon or graft, comprising a therapeutically effective amount of at least one retinoid receptors ligand as active ingredient and a coating matrix; implanting said device within an artery of a patient; and delivering the active ingredient from the device to the artery at a minimum release rate of 1 percent of the total dosage of the active ingredient on the device per day throughout an entire administration period from the time of implantation of the device until the time that all the active ingredient is released from the device.

Further preferred embodiments of the present invention are defined in dependent claim 2.

Other objects and advantages of the present invention will become apparent to those skilled in the art in view of the following detailed description.

### DETAILED DESCRIPTION OF THE INVENTION

For the purposes of the present invention, the term "retinoid receptors" refers to receptors of retinoids in general, including RAR and RXR. The term "RAR" refers to Retinoic Acid Receptor, the term "RXR" refers to Retinoid X Receptor.

The term "biodegradable polymer" means a polymer that, when implanted, degrades gradually either by being metabolized or by any other mechanism. The term "biostable or stable polymer" refers to a polymer that, when implanted, does not degrade or has a minimal degradation.

One of the main disadvantages of bare metal stents is neointimal accumulation resulting in restenosis. Drug eluting stents were originally introduced in order to address the problem of restenosis after percutaneous coronary intervention and later they have also been used in peripheral interventions. The use of drugs on the stents improved the outcomes of vascular procedures. However the problems of delayed re-endothelialization and in-stent thrombosis have not been completely solved with the existing stents.

Further, the existing drug eluting stents use antimicrotubular and/or immunosuppressing drugs that are more or less cytotoxic such as paclitaxel, sirolimus and everolimus. These drugs are suspected to be at least partially responsible for late events such as restenosis and thrombosis because of their effects on the vascular cells.

It has been surprisingly found that the object of the present invention is achieved by employing a retinoid receptor ligand which is all trans retinoid acid on the stent, and in particular in the claimed quantities.

Retinoid receptors are ligand-activated transcription factors that bind discrete elements within the regulatory regions of a growing list of target genes. This family of nuclear receptors includes the retinoid receptors. Retinoids and the receptors they bind are gaining an increasingly important role in both cardiovascular development and the response of blood vessels to injury.

Retinoids are a class of naturally occurring and synthetic compounds structurally related to retinol (vitamin A). Some retinoids with biological importance are retinol, retinal, retinlyl esters and retinoic acid. Retinoic acid (RA), is the active metabolite of vitamin A that can act as a retinoid receptor ligand. Due to the existence of double bonds in their molecules, most retinoids can exist in different stereochemical configurations, namely cis- and trans-. There are at least three isomers of RA with biological importance namely all trans-retinoic acid, 9-cis-retinoic acid and 13-cis retinoic acid.

RA isomers have been shown to influence the expression of many genes through interactions with 2 subfamilies of nuclear receptors, the retinoid acid receptors (RARs) and the retinoid X receptors (RXRs). Retinoids and more specifically retinoic acid are known to exhibit complex effects on proliferation, growth, differentiation and migration of vascular smooth muscle cells, including responses to injury. There is evidence, both clinical and molecular that implicates retinoids in vasculoproliferative disorders such as restenosis.

Retinoic acid is a substance naturally occurring in the body in small quantities in different isomers that is a potent ligand for the retinoid receptors namely retinoid acid receptor (RAR) and retinoid X receptor (RXR) and their isoforms.

Retinoic acid and its isomers, depending on the conditions, can act as antiproliferative agents without causing significant toxicity to the vascular tissue thus improving the outcome of vascular procedures.

It was found that stents coated with all trans acid in the claimed quantities and more specifically in a specific range of retinoic acid mass per area unit of the stent is effective in preventing restenosis and, at the same time, reducing the rate of possible restenosis and thrombosis.

Moreover, it has also been found that the quantity of the active drug (all trans-retinoic acid) is critical for the final outcome of the stenting and the performance of the stent, whereas non suitable quantity may have either effect in preventing restenosis or may have inverse effects, such as increased rate of restenosis.

According to the present invention, the treatment of restenosis after vascular procedures is achieved with the use of retinoid receptor ligand all trans retinoic acid. The treatment comprises the local application of retinoid receptor ligand for treatment of restenosis that occurs after vascular procedures e.g. balloon dilatation and stenting.

These substances are known to affect genes related with the proliferation of the smooth muscle cells thus contributing to the prevention of restenosis.

The local application of the active substances can be done with the use of appropriate devices e.g. catheters, stents, balloons and grafts. The active substances can be applied locally in the form of mixtures with polymers, solutions, dispersions, and gels and the compounds can exist in the forms of free molecules or ions, micelles, liposomes, or microparticles or nanoparticles that can be suitably coated. In the most preferred embodiment the local application of the active substances can be achieved by balloons or stents coated with the active substance alone or together with a polymer or mixture of polymers.

Additionally it was found that excessively high doses of all trans- retinoic acid may have reverse effects on revascularization. The above results are indicatively summarized in examples given further below.

According to another embodiment of the present invention, a process for the preparation of a drug-eluting device for preventing and treating restenosis and neointimal formation after vascular procedures and/or implantation of implants is as defined in claim 3.

The following examples illustrate preferred embodiments in accordance with the present invention without limiting the scope or spirit of the invention:

### EXAMPLES

### Reference Example 1:

Stainless steel coronary stents, 9 mm long, were coated with a mixture of all trans-retinoic acid and several combinations of bioadegradable polymers, including polylactide, polyglycolide and polycaprolactone and their copolymers.

In all these combinations the ratio of retinoic acid to polymer by weight was less than 10% and the overall dose of all trans-retinoic acid was less than 1 microgram per millimeter of stent length.

The stents were tested in a rabbit iliac artery model. Bare metal stents, stents coated only with polymer and stents coated with polymer and retinoic acid, were implanted in the iliac arteries of rabbits.

After 28 days, the animals were sacrificed and the stented parts of the arteries underwent histomorphometric analysis. The results showed in all cases that the rate of stenosis in the retinoic acid group was higher than the bare metal and the polymer groups. Indicative results are given in the Table 1 below:

**Table 1:**

| **Stent coating** | **No of stents** | **Average % stenosis after 28 days** |
|---|---|---|
| None (bare metal) | 6 | 23.1 |
| Poly DL-Lactide | 6 | 28.2 |
| Polyglycolide | 6 | 29.4 |
| Poly DL-Lactide-co-glycolide | 6 | 26.4 |
| Polv DL-Lactide + RA (0.9 ug/mm) | 6 | 40.2 |
| Polyglycolide +RA (0.9 ug/mm) | 6 | 36.4 |
| Poly DL-Lactide-co-glycolide +RA (0.9 ug/mm) | 6 | 25.9 |

From the above results it is obvious that the doses of retinoic acid used in Example 1 are not efficient in preventing restenosis and in addition they may cause higher stenosis rates than those observed in bare metal and polymer (without drug) coated stents.

### Example 2:

Stainless steel coronary stents being 9 mm long, were coated with a mixture of all trans-retinoic acid and polylactide-co-glycolide. The ratio of retinoic acid to polymer by weight was 25% and the overall dose of all trans-retinoic acid was 2.5 micrograms per millimeter of stent length. The stents were tested in a rabbit iliac artery model.

Bare metal stents, stents coated only with polymer and stents coated with polymer and retinoic acid, were implanted in the iliac arteries of rabbits. After 28 days, the animals were sacrificed and the stented parts of the arteries underwent histomorphometric analysis. The results showed that the percent stenosis in the retinoic acid group was lower than the bare metal and the polymer groups as given in the Table 2 below:

**Table 2:**

| **Stent coating** | **No of stents** | **Average % stenosis after 28 days** |
|---|---|---|
| None (bare metal, stainless steel) | 6 | 24.8 |
| Poly DL-Lactide-co-plycolide | 6 | 28.2 |
| Poly DL-Lactide-co-glycolide +RA (2.5 ug/mm) | 6 | 15.9 |

From the above results it is obvious that the doses of retinoic acid described in Example 2 are very efficient in preventing restenosis and present lower stenosis rates than those observed in bare metal and polymer (without drug) coated stents, in the above mentioned experimental model.

The present invention has specific advantages compared to the existing art. These advantages include effectiveness and safety, economy in materials and production and improved production method.

More specifically, it is known to those skilled in the art that retinoids, as substances naturally occurring in the body, can control proliferation without being cytotoxic thus reducing or eliminating pitfalls of existing devices such as excessive inflammation, late stenosis and thrombosis.

Additionally retinoic acid as a drug is less expensive than synthetic drugs (such as for example everolimus) that are used in existing drug eluting stents. Finally a simple and economic production method is provided that reduces the overall cost of the production.

## Claims

1. Drug eluting device for preventing and treating restenosis and neointimal formation after vascular procedures and/or implantation of implants comprising an implantable and non-implantable stent, wherein said device comprises a therapeutically effective amount of at least one retinoid receptors ligand as active ingredient and a coating matrix, wherein said device is coated with a mixture of all trans- retinoic acid and polylactide-co-glycolide, wherein the ratio of retinoic acid polymer by weight is 25% and the overall dose of all trans-retinoic acid is 2.5 micrograms per millimeter of stent length.

2. The device according to claim 1, wherein said device is a drug eluting vascular, coronary or peripheral or renal stent, that is made of cobalt chromium, or stainless steel, or a memory-responsive metal, or other metal alloy, or a polymer or a blend of polymers.

3. A process for the preparation of a drug eluting device for preventing and treating restenosis and neointimal formation after vascular procedures and/or implantation of implants comprising an implantable and non-implantable stent, wherein said device comprises a therapeutically effective amount of at least one retinoid receptors ligand as active ingredient and a coating matrix, wherein said device is coated with a mixture of all trans- retinoic acid and polylactide-co-glycolide, wherein the ratio of retinoic acid polymer by weight is 25% and the overall dose of all trans-retinoic acid is 2.5 micrograms per millimeter of stent length, said process comprises the following steps:
- Preparation of a first mixture of polymers and dilution or dispersion of this blend in an appropriate solvent;
- Preparation of a second mixture of a solution of all trans- retinoic acid in an appropriate solvent;
- Application of the first and second mixtures on the surface of the stent, with a suitable method, including but not limited to immersion and spraying, preferably microspraying, in one or more steps, in a single or successive layers; and
- Removal of possible remaining solvents with evaporation.

## Patentansprüche

1. Arzneimitteleluierende Vorrichtung zum Verhindern und zur Behandlung von Restenose und Neointimabildung nach Gefäßoperationen und/oder Implantation von einen implantierbaren und nichtimplantierbaren Stent umfassenden Implantaten, wobei die Vorrichtung eine therapeutisch wirksame Menge mindestens eines Retinoidrezeptorliganden als Wirkstoff und eine Überzugsmatrix umfasst, wobei die Vorrichtung mit einer Mischung von all-trans-Retinsäure und Polylactid-coglycolid überzogen ist, wobei das Gewichtsverhältnis von Retinsäurepolymer 25% beträgt und die Gesamtdosis von all-trans-Retinsäure 2,5 Mikrogramm pro Millimeter Stentlänge beträgt.

2. Vorrichtung nach Anspruch 1, wobei es sich bei der Vorrichtung um einen arzneimitteleluierenden vaskulären, koronaren oder peripheren oder renalen Stent aus Cobaltchrom oder Edelstahl oder einem gedächtnisreaktiven Metall oder einer anderen Metalllegierung oder einem Polymer oder einer Polymermischung handelt.

3. Verfahren zur Herstellung einer arzneimitteleluierenden Vorrichtung zum Verhindern und zur Behandlung von Restenose und Neointimabildung nach Gefäßoperationen und/oder Implantation von einen implantierbaren und nichtimplantierbaren Stent umfassenden Implantaten, wobei die Vorrichtung eine therapeutisch wirksame Menge mindestens eines Retinoidrezeptorliganden als Wirkstoff und eine Überzugsmatrix umfasst, wobei die Vorrichtung mit einer Mischung von all-trans-Retinsäure und Polylactid-coglycolid überzogen ist, wobei das Gewichtsverhältnis von Retinsäurepolymer 25% beträgt und die Gesamtdosis von all-trans-Retinsäure 2,5 Mikrogramm pro Millimeter Stentlänge beträgt, wobei das Verfahren die folgenden Schritte umfasst:
- die Herstellung einer ersten Mischung von Polymeren und die Verdünnung oder das Dispergieren dieser Mischung in einem geeigneten Lösungsmittel,
- die Herstellung einer zweiten Mischung einer Lösung von all-trans-Retinsäure in einem geeigneten Lösungsmittel,
- das Aufbringen der ersten und der zweiten Mischung auf der Oberfläche des Stents mit einem geeigneten Verfahren einschließlich, jedoch nicht darauf beschränkt, Eintauchen und Sprühen, vorzugsweise Mikrosprühen, in einem oder mehreren Schritten, in einer einzelnen oder aufeinanderfolgenden Schichten, und
- das Entfernen möglicher Lösungsmittelreste durch Abdampfen.

## Revendications

1. Dispositif d'élution de médicament pour la prévention et le traitement d'une resténose et de formation néointimale après des interventions vasculaires et/ou l'implantation d'implants comprenant un stent implantable ou non implantable, ledit dispositif comprenant une quantité thérapeutiquement efficace d'au moins un ligand des récepteurs rétinoïdes en tant qu'ingrédient actif et une matrice de revêtement, ledit dispositif étant revêtu avec un mélange d'acide tout-trans-rétinoique et d'un polylactide-co-glycolide, le rapport d'acide rétinoïque sur polymère en poids étant de 25 % et la dose totale d'acide tout-trans-rétinoique étant de 2,5 microgrammes par millimètre de longueur de stent.

2. Dispositif selon la revendication 1, ledit dispositif étant un stent vasculaire, coronarien ou périphérique ou rénal d'élution de médicament, qui est composé de cobalt chrome, ou d'acier inoxydable, ou d'un métal à mémoire de forme, ou d'un autre alliage métallique, ou d'un polymère ou d'un mélange de polymères.

3. Procédé pour la préparation d'un dispositif d'élution de médicament pour la prévention et le traitement d'une resténose et de formation néointimale après des interventions vasculaires et/ou l'implantation d'implants comprenant un stent implantable ou non implantable, ledit dispositif comprenant une quantité thérapeutiquement efficace d'au moins un ligand des récepteurs rétinoïdes en tant qu'ingrédient actif et une matrice de revêtement, ledit dispositif étant revêtu avec un mélange d'acide tout-trans-rétinoique et d'un polylactide-co-glycolide, le rapport d'acide rétinoïque sur polymère en poids étant de 25 % et la dose totale d'acide tout-trans-rétinoïque étant de 2,5 microgrammes par millimètre de longueur de stent, ledit procédé comprenant les étapes suivantes :
- préparation d'un premier mélange de polymères et dilution ou dispersion de ce mélange dans un solvant approprié ;
- préparation d'un deuxième mélange d'une solution d'acide tout-trans-rétinoïque dans un solvant approprié ;
- application des premier et deuxième mélanges sur la surface du stent, avec un procédé approprié, comportant, mais ne s'y limitant pas, une immersion et une pulvérisation, préférablement une micropulvérisation, en une unique couche ou en des couches successives ; et
- élimination de solvants restants possibles par évaporation.
